(19) Europäisches Patentamt
European Patent Office
Office européen des brevets

(11) Numéro de publication: **0 270 468 B1**

(12) **FASCICULE DE BREVET EUROPEEN**

(45) Date de publication de fascicule du brevet: **24.07.91**

(51) Int. Cl.[5]: **C07C 49/403**, C07C 35/08, C07C 27/12, C07C 29/48, C07C 45/33, C07C 45/53

(21) Numéro de dépôt: **87420322.7**

(22) Date de dépôt: **30.11.87**

Le dossier contient des informations techniques présentées postérieurement au dépôt de la demande et ne figurant pas dans le présent fascicule.

(54) **Procédé de préparation d'un mélange renfermant un cyclohexanol et de la cyclohexanone à partir du cyclohexane.**

(30) Priorité: **05.12.86 FR 8617249**

(43) Date de publication de la demande: **08.06.88 Bulletin 88/23**

(45) Mention de la délivrance du brevet: **24.07.91 Bulletin 91/30**

(84) Etats contractants désignés: **BE DE ES FR GB IT NL**

(56) Documents cités:
**EP-A- 0 027 937**
**EP-A- 0 157 657**
**GB-A- 1 191 573**

(73) Titulaire: **RHONE-POULENC CHIMIE**
**25, quai Paul Doumer**
**F-92408 Courbevoie Cédex(FR)**

(72) Inventeur: **Igersheim, Françoise**
**21, rue Florian**
**F-69100 Villeurbanne(FR)**
Inventeur: **Costantini, Michel**
**10, rue du Docteur Bonhomme**
**F-69003 Lyon(FR)**

(74) Mandataire: **Varnière-Grange, Monique et al**
**RHONE-POULENC INTERSERVICES Service Brevets Chimie Centre de Recherches des Carrières B.P. 62**
**F-69192 Saint-Fons Cédex(FR)**

**EP 0 270 468 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention est relative à un procédé de préparation d'un mélange refermant du cyclohexanol et de la cyclohexanone à partir du cyclohexane.

Elle concerne plus particulièrement le stade de décomposition d'un procédé dans lequel le cyclohexane est oxydé en présence d'air pour former un mélange renfermant de l'hydroperoxyde de cyclohexyle désigné dans ce qui suit par HPOCH, cet hydroperoxyde étant alors décomposé en présence de cyclohexane. Les produits principalement formés lors de la décomposition sont le cyclohexanol (OL) et la cyclohexanone (ONE). Ces produits sont convertis par oxydation en acide adipique, une des matières premières des polyamides. La cyclohexanone est aussi un intermédiaire du caprolactame, produit qui entre dans la fabrication de certains polyamides.

La présente invention vise plus particulièrement à augmenter la conversion globale du cyclohexane par un accroissement de sa participation, au stade de décomposition de l'HPOCH, à la formation de produits d'oxydation utiles, la réaction idéale pouvant être représentée par l'équation réactionnelle (1) ci-après :

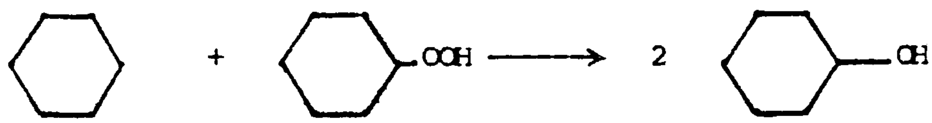 (1)

D'autres réactions de décomposition de l'HPOCH peuvent être répresentées par les équations (2) et (3) ci-après :

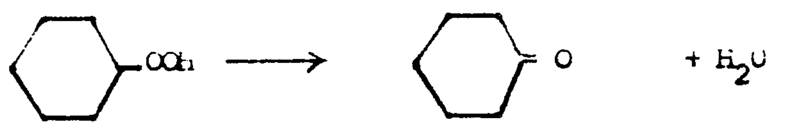 (2)

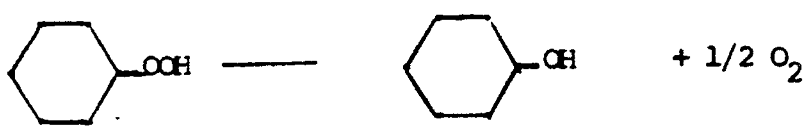 (3)

A.M. TRZECIAK et Coll. dans J. Mol. Cat.10,69, 1980 ont décrit l'utilisation de sels de ruthénium comme catalyseur de dépéroxydation de l'hydroperoxyde de cumyle. Toutefois, le pouvoir oxydant de l'hydroperoxyde n'est pas utilisé pour un oxyder substrat.

Le brevet français 1 547 427 décrit l'utilisation de sels solubles de ruthénium pour catalyser la déperoxydation de l'HPOCH dans le cyclohexane. Le rendement apparent en cyclohexanone et cyclohexanol c'est à dire le rapport du nombre de moles de cyclohexanone et de cyclohexanol formées au nombre de moles d' hydroperoxyde de cyclohexyle transformées, reste faible et la concentration en ruthénium par rapport à l'HPOCH est relativement importante en pratique.

Il a maintenant été trouvé un procédé permettant d'améliorer la participation du cyclohexane au stade de décomposition de l'HPOCH en présence de catalyseurs à base de ruthénium.

La présente invention a donc pour objet un procédé de production d'un mélange de cyclohexanol et de cyclohexanone dans lequel on oxyde le cyclohexane par l'air pour produire un mélange contenant de l'hydroperoxyde de cyclohexyle et dans lequel l'hydroperoxyde de cyclohexyle est décomposé en présence de cyclohexane pour former le cyclohexanol et la cyclohexanone en présence de composés solubles de ruthénium, procédé qui comprend la décomposition de l'hydroperoxyde de cyclohexyle par la mise en contact, à une température comprise entre 20 et 140 °C d'un mélange réactionnel contenant du cyclohexane et de l'ordre de 0,1 à 10 % en poids d'hydroperoxyde de cyclohexyle en présence d'une quantité d'un composé du ruthénium soluble dans le milieu réactionnel et n'excédant pas 500 ppm de ruthénium par

rapport au dit hydroperoxyde et d'un coordinat répondant à la formule générale :

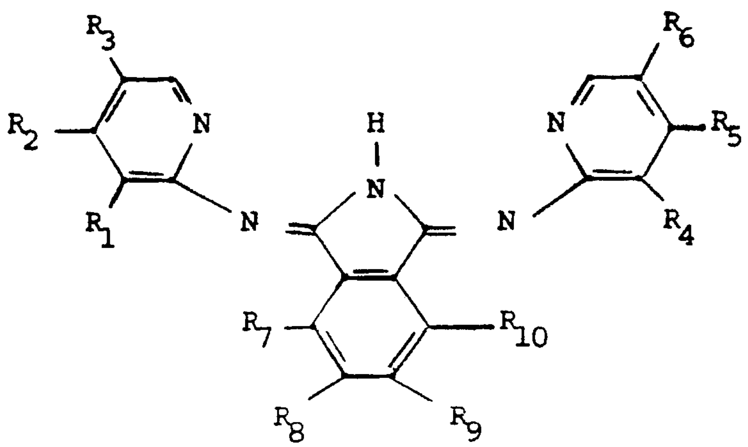

dans laquelle :

. $R_1$ à $R_6$, identiques ou différents, représentent :
  . un atome d'hydrogène,
  . un radical alkyle ayant au maximum 6 atomes de carbone,
  . un radical alcoxy ayant de 2 à 6 atomes de carbone,
  . un radical phényle, benzyle ou phénéthyle,
  . deux quelconques des radicaux portés par des atomes de carbone adjacents pouvant former ensemble un radical unique comportant quatre entités CH formant un noyau benzènique condensé avec le cycle de la pyridine.

. $R_7$ à $R_{10}$, identiques ou différents représentent :
  . un atome d'hydrogène,
  . un radical alkyle ayant au maximum 6 atomes de carbone,
  . un radical alcoxy ayant de 2 à 6 atomes de carbone,
  . deux quelconques des radicaux portés par des atomes de carbone adjacents pouvant former ensemble un radical unique comportant quatre entités CH formant un noyau benzènique condensé avec le cycle de l'isoindoline.

Ainsi la mise en oeuvre du procédé selon l'invention nécessite la présence d'une quantité catalytiquement efficace d'un composé soluble du ruthénium.

Par quantité catalytiquement efficace on entend une teneur suffisante pour donner une vitesse de réaction industriellement acceptable, vitesse qui dépend également des autres conditions de la réaction, tout en n'alourdissant pas les opérations de recyclage et/ou de purification des produits visés.

En général la teneur en ruthénium est d'au moins 1 ppm par rapport à l'HPOCH. Pour une bonne mise en oeuvre de l'invention, elle est comprise entre 2 et 250 ppm par rapport à l'HPOCH.

Par composés du ruthénium solubles dans le milieu réactionnel on entend essentiellement ceux qui présentent une solubilité dans le cyclohexane d'au moins 0,1g/litre à la température de 25° C. A titre d'exemples de tels composés on peut citer le naphténate, l'hexanoate, l'octoate, le stéarate, les dérivés carbonyles, les formes chélatées ainsi que les esters (de l'acide) des acides dérivé(s) du ruthénium. On recourt avantageusement au naphténate, à l'octoate ou à l'acétylacétonate de ruthénium. Ce dernier convient plus particulièrement bien à la mise en oeuvre du présent procédé.

La mise en oeuvre du présent procédé requiert la présence d'un coordinat choisi parmi les bis(pyridyl-2 imino)-1,3 isoindolines de formule générale indiquée ci-avant.

Plus spécifiquement :

. $R_1$ à $R_6$, identiques ou différents, représentent :
  . un atome d'hydrogène,
  . un radical méthyle,
  . un radical tertiobutyle,
  . un radical méthoxy,
  . un radical tertiobutoxy.

. $R_7$ à $R_{10}$, identiques ou différents représentent :

. un atome d'hydrogène,
. un radical méthyle,
. un radical tertiobutyle.

De manière avantageuse l'un au moins des radicaux $R_1$ à $R_3$ et l'un au moins des radicaux $R_4$ à $R_6$ sont des atomes d'hydrogène,

De préférence deux au moins des radicaux $R_1$ à $R_3$ et deux au moins des radicaux $R_4$ à $R_6$ sont des atomes d'hydrogène.

De manière avantageuse, l'un des radicaux $R_1$ à $R_3$ et l'un des radicaux $R_4$ à $R_6$ représentent un groupe méthyle ou, $R_2$ et $R_5$ représentent un groupe tertiobutyle, et les autres radicaux $R_1$ à $R_6$ représentent des atomes d'hydrogène.

A titre d'exemples de bis(pyridyl-2 imino)-1,3 isoindolines utilisables dans le cadre du présent procédé on peut citer :

- la bis(pyridyl-2 imino)-1,3 isoindoline,
- la bis(méthyl-3 pyridyl-2 imino)-1,3 isoindoline, ses isomères et leurs mélanges,
- la bis(diméthyl-3,4 pyridyl-2 imino)-1,3 isoindoline,
- la bis(éthyl-3 pyridyl-2 imino)-1,3 isoindoline,
- la bis(butyl-3 pyridyl-2 imino)-1,3 isoindoline,
- la bis(pentyl-4 pyridyl-2 imino)-1,3 isoindoline,
- la bis(isohexyl-4 pyridyl-2 imino)-1,3 isoindoline,
- la bis(phényl-5 pyridyl-2 imino)-1,3 isoindoline,
- la (tertiobutyl-5) bis(pyridyl-2 imino)-1,3 isoindoline,
- la (diméthyl-4,7) bis(méthyl-3 pyridyl-2 imino)-1,3 isoindoline,
- la tétrakis(tertiopentyl)-4,5,6,7 bis (pyridyl-2 imino)-1,3 isoindoline,
- la (triméthyl-4,5,6) bis(pyridyl-2 imino)-1,3 isoindoline,
- l'hexyl-4 bis(pyridyl-2 imino)-1,3 isoindoline et,
- la bis(isoquinolyle-1 imino)-1,3 isoindoline et,

En général, la quantité de coordinat est comprise entre 1 et 10 mole par atome-gramme de ruthénium présent dans le milieu réactionnel. Pour une bonne mise en oeuvre de l'invention cette quantité sera comprise entre 1,5 et 2,5 moles par atome-gramme de ruthénium et de préférence de l'ordre de 2 moles par atome-gramme de ruthénium.

Il est important de souligner que, compte-tenu des quantités extrêmement faibles de ruthénium mises en oeuvre dans le cadre du présent procédé, les quantités de coordinat engagées dans le contexte de la présente invention, sont elles aussi extrêmement faibles, ce qui constitue un avantage supplémentaire du présent procédé.

En général, la quantité de coordinat est telle que le rapport atomique N/Ru soit compris entre 5 et 50 et, de préférence entre 5 et 15. Pour une bonne mise en oeuvre de l'invention ce rapport sera de l'ordre de 10.

La réaction de décomposition est conduite en phase liquide, la concentration de l'hydroperoxyde de cyclohexyle étant comprise entre 0,1 et 10 % en poids. Cette concentration est avantageusement comprise entre 0,5 et 8 %.

On peut faire appel à divers solvants tels les alcanes parmi lesquels on citera plus particulièrement l'hexane, l'heptane et l'isooctane ; les cycloalcanes parmi lesquels on mentionnera à titre illustratif le cyclohexane et le cyclooctane, les hydrocarbures aromatiques tels le benzène, le toluène et le xylène et leurs mélanges.

Toutefois il est à noter que l'hydroperoxyde de cyclohexyle étant produit sous la forme d'une solution dans le cyclohexane par oxydation comme cela a été indiqué ci-avant, la réaction de décomposition est avantageusement réalisée sur une solution provenant de l'oxydation du cyclohexane dans laquelle la concentration en hydroperoxyde de cyclohexyle est comprise dans les limites indiquées précédemment. Cette solution peut être utilisée en l'état ou après élimination de certains constituants de manière en soi connue. Il est également possible d'utiliser une solution d'hydroperoxyde de cyclohexyle dans le cyclohexane sensiblement pur.

La température est généralement comprise entre 20 et 140 °C et, de préférence, entre 80 et 100 °C.

La pression atmosphérique ou supérieure à la pression atmosphérique sera suffisante pour maintenir le cyclohexane en phase liquide.

La durée de réaction (ou le temps de séjour) est généralement compris entre quelques minutes et 4 heures et peut être ajustée compte tenu des objectifs de production, de la quantité des divers constituants du système catalytique engagée et des autres paramètres de la réaction.

En fin de réaction les produits peuvent être récupérés par tout moyen approprié, par exemple par distillation.

Les exemples ci-après illustrent la présente invention.

Dans ces exemples, RT (OLONE) désigne le rendement apparent en cyclohexanone et cyclohexanol c'est à dire le rapport du nombre de moles de cyclohexanone et de cyclohexanol formées au nombre de moles d'hydroperoxyde de cyclohexyle transformées. Un rendement supérieur à 100 % indique qu'une fraction des produits en cause provient de l'oxydation du cyclohexane.

EXEMPLE 1:

Dans un réacteur muni d'un agitateur central et d'un réfrigérant, on charge sous atmosphère inerte :

- 84 g (1 mole) de cyclohexane,
- 0,88 mg (2,1 $10^{-3}$ mmol) d'acétylacétonate de ruthénium III,
- 1,4 mg (4,2 $10^{-3}$ mmol) de bis(méthyl-3 pyridyl-2 imino)-1,3 isoindoline.

Le mélange est chauffé à 80 °C et lorsque cette température est atteinte on ajoute le plus rapidement possible (en environ 1 minute):

- 6 g d'hydroperoxyde de cyclohexyle à 97 %.

L'agitation est maintenue jusqu'à ce que la teneur en oxygène peroxydique soit nulle ou stable (soit 1 heure dans le cas présent). Cette durée est appelée temps de réaction et est désignée par t, dans ce qui suit. Le mélange est refroidi. Le mélange est ensuite analysé par chromatographie en phase vapeur.

Il contient :

Cyclohexanol : 3 610 mg soit 36,8 mmol
Cyclohexanone : 2 210 mg soit 20,6 mmol
Ce qui correspond à :
RT (OLONE) 116,5 %

EXEMPLES 2 à 4 : ESSAI TEMOIN (a):

Une première série d'essais est réalisée selon le mode opératoire décrit pour l'exemple 1 ci-avant ; les conditions particulières ainsi que les résultats obtenus étant rassemblés dans le tableau I ci-après

Tableau I

| N° Essai | Ru mmol | B P I mmol | t | OL mmol | ONE mmol | RT (%) OLONE |
|---|---|---|---|---|---|---|
| 1 | $2,1.10^{-3}$ | $4,2.10^{-3}$ | 1h | 36,8 | 22,5 | 116,5 |
| 2 | ,, | $6,3.10^{-3}$ | 1h | 34,6 | 24,0 | 117 |
| 3 | ,, | $2,1.10^{-3}$ | 3h | 34,6 | 24,0 | 117 |
| 4 | $210.10^{-3}$ | $420.10^{-3}$ | NB | 31,1 | 24,5 | 111 |
| a | $2,1.10^{-3}$ | 0 | 6h | 35,0 | 24,5 | 117 |

NB : la décomposition de l'HPOCH est instantanée

B P I : bis(méthyl-3 pyridyl-2 imino)-1,3 isoindoline

EXEMPLE 5 à 10 :

Une seconde série d'essais est réalisée dans l'appareillage et selon un mode opératoire analogue à celui décrit pour l'exemple 1, à ceci 1 `ex1e 1, à ceci près que la température, différente, est désignée par T dans ce qui suit et que le taux de transformation de l'HPOCH (désigné par TT dans ce qui suit) n'est pas nécessairement 100% au bout de la durée de la réaction désignée par t. Les conditions particulières ainsi que les résultats obtenus sont rassemblés dans le tableau II ci-après.

**Tableau II**

| N° Essai | Ru mmol | B P I mmol | T (°C) | t | TT | RT (%) OLONE | OL/ONE (*) |
|---|---|---|---|---|---|---|---|
| 1 | $2,1.10^{-3}$ | $4,2.10^{-3}$ | 80 | 1h | 100 | 116,5 | 1,6 |
| 5 | ,, | ,, | 100 | 30mn | 93 | 117 | 2 |
| 6 | $1,05.10^{-3}$ | $2,1.10^{-3}$ | 100 | 1h | 94 | 117 | 2 |
| 7 | $1,05.10^{-3}$ | $4,2.10^{-3}$ | 100 | 1h | 90 | 117,5 | 2 |
| 8 | $2,1.10^{-3}$ | $4,2.10^{-3}$ | 120 | 15mn | 100 | 114 | 2,2 |
| 9 | ,, | ,, | 140 | 10mn | 100 | 113 | 2,3 |
| 10 | ,, | ,, | 20 | 5h | 100 | 106 | 1,2 |

(*) : Rapport molaire cyclohexanol/cyclohexanone

B P I : bis(méthyl-3 pyridyl-2 imino)-1,3 isoindoline

## Revendications

1. Procédé de production d'un mélange de cyclohexanol et de cyclohexanone dans lequel on oxyde le cyclohexane par l'air pour produire un mélange contenant de l'hydroperoxyde de cyclohexyle et dans lequel l'hydroperoxyde de cyclohexyle est décomposé en présence de cyclohexane pour former le cyclohexanol et la cyclohexanone, procédé qui comprend la décomposition de l'hydroperoxyde de cyclohexyle par la mise en contact, à une température comprise entre 20 et 140° C d'un mélange réactionnel contenant du cyclohexane et de l'ordre de 0,1 à 10 % en poids d hydroperoxyde de cyclohexyle en présence d'un composé du ruthénium soluble dans le milieu réactionnel, caractérisé en ce que la quantité de ruthénium n'excède au dit hydroperoxyde et par la présence d'un d'un coordinat répondant à la formule générale :

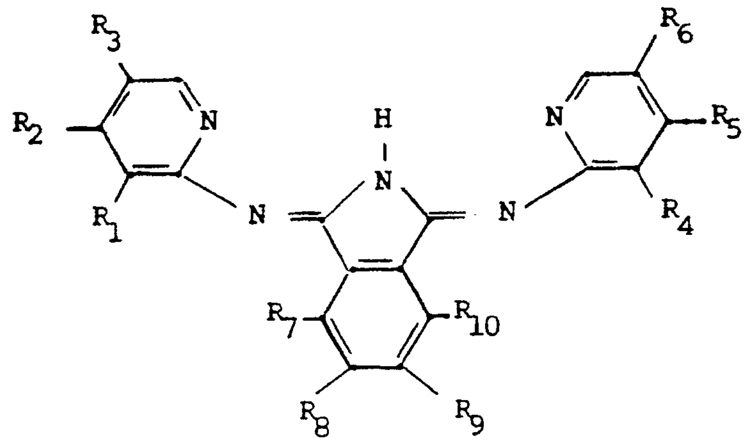

dans laquelle :

. $R_1$ à $R_6$, identiques ou différents, représentent :

. un atome d'hydrogène,

. un radical alkyle ayant au maximum 6 atomes de carbone,

. un radical alcoxy ayant de 2 à 6 atomes de carbone,

. un radical phényle, benzyle ou phénéthyle,

. deux quelconques des radicaux portés par des atomes de carbone adjacents pouvant former ensemble un radical unique comportant quatre entités CH formant un noyau benzénique condensé avec le cycle de la pyridine.

. $R_7$ à $R_{10}$, identiques ou différents représentent :

. un atome d'hydrogène,

. un radical alkyle ayant au maximum 6 atomes de carbone,

. un radical alcoxy ayant de 2 à 6 atomes de carbone,

. deux quelconques des radicaux portés par des atomes de carbone adjacents pouvant former ensemble un radical unique comportant quatre entités CH formant un noyau benzénique condensé avec le cycle de l'isoindoline.

**2.** Procédé selon la revendication 1, caractérisé en ce que la concentration du ruthénium est comprise entre 2 et 250 ppm par rapport à l'hydroperoxyde de cyclohexyle.

**3.** Procédé selon l'une quelconque des revendications précédentes, caractérisé en ce que la quantité de coordinat est comprise entre 1 et 10 moles par atome-gramme de ruthénium.

**4.** Procédé selon la revendication 3, caractérisé en ce que la quantité de coordinat est comprise entre 1,5 et 2,5 moles par atome-gramme de ruthénium.

**5.** Procédé selon l'une quelconque des revendications précédentes, caractérisé en ce que le coordinat répond à la formule figurant dans la revendication dans laquelle :

. $R_1$ à $R_6$, identiques ou différents, représentent :

. un atome d'hydrogène,

. un radical méthyle,

. un radical tertiobutyle,

. un radical méthoxy,

. un radical tertiobutoxy.

. $R_7$ à $R_{10}$, identiques ou différents représentent :

. un atome d'hydrogène,

. un radical méthyle,

. un radical tertiobutyle.

**6.** Procédé selon l'une quelconque des revendications précédentes, caractérisé en ce que le coordinat répond à la formule donnée dans la revendication 1 dans laquelle l'un au moins des radicaux $R_1$ à $R_3$ et l'un au moins des radicaux $R_4$ à $R_6$ sont des atomes d'hydrogène.

7. Procédé selon l'une quelconque des revendications précédentes, caractérisé en ce que le coordinat répond à la formule donnée dans la revendication 1 dans laquelle deux au moins des radicaux $R_1$ à $R_3$ et deux au moins des radicaux $R_4$ à $R_6$ sont des atomes d'hydrogène.

8. Procédé selon l'une quelconque des revendications précédentes, caractérisé en ce que le coordinat répond à la formule donnée dans la revendication 1 dans laquelle l'un des radicaux $R_1$ à $R_3$ et l'un des radicaux $R_4$ à $R_6$ représentent un groupe méthyle ou, $R_2$ et $R_5$ représentent un groupe tertiobutyle, et les autres radicaux $R_1$ et $R_6$ représentent des atomes d'hydrogène.

9. Procédé selon la revendication 8, caractérisé en ce que le coordinat est choisi parmi la bis(pyridyl-2 imino)-1,3 isoindoline et la bis(méthyl-3 pyridyl-2 imino)-1,3 isoindoline.

10. Procédé selon l'une quelconque des revendications caractérisé en ce que le ruthénium est introduit dans le milieu réactionnel sous forme d'un composé choisi parmi le naphénate, l'octoate, l'hexanoate et l'acétylacétonate de ruthénium.

11. Procédé selon la revendication 10, caractérisé en ce que le ruthénium est introduit dans le milieu réactionnel sous forme de l'acétylacétonate de ruthénium.

12. Procédé selon l'une quelconque des revendications précédentes, caractérisé en ce que la décomposition est conduite à une température comprise entre 80 et 100 ° C.

13. Procédé selon l'une quelconque des revendications précédentes, caractérisé en ce qu'au stade de la décomposition, la concentration de l'hydroperoxyde de cyclohexyle est comprise entre 0,5 et 8 % en poids.

**Claims**

1. Process for producing a mixture of cyclohexanol and cyclohexanone in which cyclohexane is oxidised by air to produce a mixture containing cyclohexyl hydroperoxide and in which the cyclohexyl hydroperoxide is decomposed in the presence of cyclohexane to form cyclohexanol and cyclohexanone, which process comprises the decomposition of the cyclohexyl hydroperoxide by bringing into contact, at a temperature of between 20 and 140 ° C, a reaction mixture containing cyclohexane and of the order of 0.1 to 10% by weight of cyclohexyl hydroperoxide in the presence of a ruthenium compound which is soluble in the reaction medium, characterised in that the quantity of ruthenium does not exceed 500 ppm with respect to the said hydroperoxide, and by the presence of a ligand corresponding to the general formula:

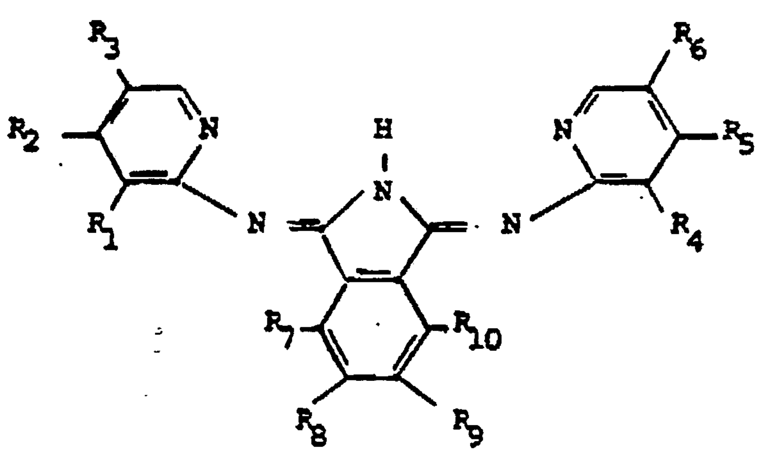

in which:
$R_1$ to $R_6$, which may be identical or different, denote:
. a hydrogen atom,
. an alkyl radical having a maximum of 6 carbon atoms,

. an alkoxy radical having from 2 to 6 carbon atoms,
. a phenyl, benzyl or phenethyl radical,
. any two of the radicals borne by adjacent carbon atoms being capable of forming together a single radical containing four CH entities forming a benzene ring fused with the pyridine ring.

$R_7$ to $R_{10}$, which may be identical or different, denote:
. a hydrogen atom,
. an alkyl radical having a maximum of 6 carbon atoms,
. an alkoxy radical having from 2 to 6 carbon atoms,
. any two of the radicals borne by adjacent carbon atoms being capable of forming together a single radical containing four CH entities forming a benzene ring fused with the isoindoline ring-system.

**2.** Process according to Claim 1, characterised in that the concentration of ruthenium is between 2 and 250 ppm with respect to the cyclohexyl hydroperoxide.

**3.** Process according to any one of the preceding claims, characterised in that the quantity of ligand is between 1 and 10 moles per gram-atom of ruthenium.

**4.** Process according to Claim 3, characterised in that the quantity of ligand is between 1.5 and 2.5 moles per gram-atom of ruthenium.

**5.** Process according to any one of the preceding claims, characterised in that the ligand corresponds to the formula appearing in Claim 1, in which:
R, to $R_6$, which may be identical or different, denote:
. a hydrogen atom,
. a methyl radical,
. a tert-butyl radical,
. a methoxy radical,
. a tert-butoxy radical.

$R_7$ to $R_{10}$, which may be identical or different, denote:
. a hydrogen atom,
. a methyl radical,
. a tert-butyl radical.

**6.** Process according to any one of the preceding claims, characterised in that the ligand corresponds to the formula given in Claim 1, in which at least one of the radicals $R_1$, to $R_3$ and at least one of the radicals $R_4$ to $R_6$ are hydrogen atoms.

**7.** Process according to any one of the preceding claims, characterised in that the ligand corresponds to the formula given in Claim 1, in which at least two of the radicals $R_1$, to $R_3$ and at least two of the radicals $R_4$ to $R_6$ are hydrogen atoms.

**8.** Process according to any one of the preceding claims, characterised in that the ligand corresponds to the formula given in Claim 1, in which one of the radicals $R_1$ to $R_9$ and one of the radicals $R_4$ to $R_6$ denote a methyl group, or $R_2$ and $R_5$ denote a tert-butyl group and the other radicals $R_1$, to $R_6$ denote hydrogen atoms.

**9.** Process according to Claim 8, characterised in that the ligand is chosen from 1,3-bis(2-pyridylimino)-isoindoline and 1,3-bis(3-methyl-2-pyridylimino)-isoindoline.

**10.** Process according to any one of the preceding claims, characterised in that the ruthenium is introduced into the reaction medium in the form of a compound chosen from ruthenium naphthenate, octoate, hexanoate and acetylacetonate.

**11.** Process according to Claim 10, characterised in that the ruthenium is introduced into the reaction medium in the form of ruthenium acetylacetonate.

**12.** Process according to any one of the preceding claims, characterised in that the decomposition is

performed at a temperature of between 80 and 100°C.

13. Process according to any one of the preceding claims, characterised in that, at the decomposition stage, the concentration of cyclohexyl hydroperoxide is between 0.5 and 8% by weight.

**Patentansprüche**

1. Verfahren zur Herstellung eines Gemisches aus Cyclohexanol und Cyclohexanon, bei dem man Cyclohexan mit Luft oxidiert, um ein Gemisch herzustellen, enthaltend Cyclohexyl-hydroperoxid, und bei dem das Cyclohexyl-hydroperoxid zersetzt wird in Gegenwart von Cyclohexan unter Bildung von Cyclohexanol und Cyclohexanon, wobei das Verfahren umfaßt die Zersetzung des Cyclohexyl-hydroperoxids durch Zusammenbringen bei einer Temperatur zwischen 20 und 140°C eines Reaktionsgemisches, enthaltend Cyclohexan und 0,1 bis 10 Gew.-% Cyclohexyl-hydroperoxid, in Gegenwart einer in dem Reaktionsmedium löslichen Verbindung von Ruthenium, dadurch gekennzeichnet, daß die Menge an Ruthenium nicht über 500 ppm, bezogen auf das Hydroperoxid, hinausgeht und in Gegenwart eines Liganden entsprechend der allgemeinen Formel:

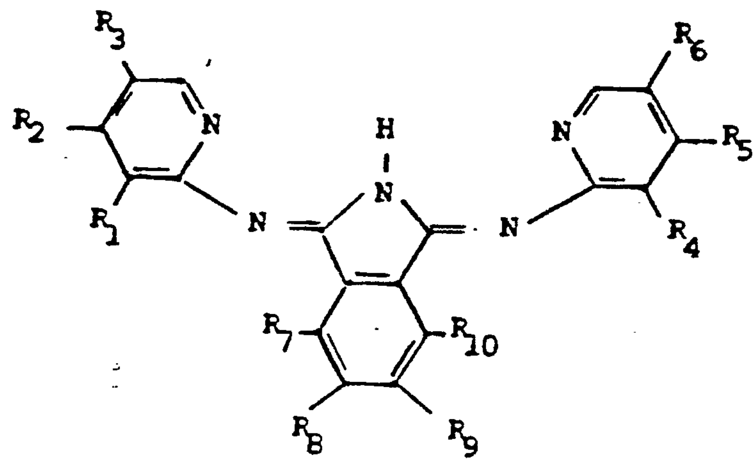

in der:
$R_1$ bis $R_6$ gleich oder verschieden sind und bedeuten:
- ein Wasserstoffatom,
- einen Alkylrest mit höchstens 6 Kohlenstoffatomen,
- einen Alkoxyrest mit 2 bis 6 Kohlenstoffatomen,
- einen Phenyl-, Benzyl- oder Phenethylrest,
- zwei der Reste, die an benachbarten Kohlenstoffatomen vorhanden sind, zusammen einen einzigen Rest mit vier CH-Gruppen bilden können, die einen Benzolring bilden, der mit dem Pyridinring kondensiert ist;

$R_7$ bis $R_{10}$ gleich oder verschieden sind und bedeuten:
- ein Wasserstoffatom,
- einen Alkylrest mit höchstens 6 Kohlenstoffatomen,
- einen Alkoxyrest mit 2 bis 6 Kohlenstoffatomen,
- wobei zwei der Reste, die an benachbarten Kohlenstoffatomen vorhanden sind, zusammen einen einzigen Rest mit vier CH-Gruppen bilden können, die einen Benzolring bilden, der mit dem Isoindolinring kondensiert ist.

2. Verfahren nach Anspruch 1, dadurch gekennzeichnet, daß die Konzentration an Ruthenium zwischen 2 und 250 ppm, bezogen auf das Cyclohexyl-hydroperoxid, beträgt.

3. Verfahren nach einem der vorangehenden Ansprüche, dadurch gekennzeichnet, daß die Menge des Liganden zwischen 1 und 10 mol pro gAtom Ruthenium beträgt.

4. Verfahren nach Anspruch 3, dadurch gekennzeichnet, daß die Menge an Liganden zwischen 1,5 und 2,5 mol pro gAtom Ruthenium beträgt.

5. Verfahren nach einem der vorangehenden Ansprüche, dadurch gekennzeichnet, daß der Ligand der in Anspruch (1) angegebenen Formel entspricht, bei der
$R_1$ bis $R_6$ gleich oder verschieden sind und bedeuten:
- ein Wasserstoffatom,
- eine Methylgruppe,
- eine tert.Butylgruppe,
- eine Methoxygruppe,
- eine tert.Butoxygruppe;

$R_7$ bis $R_{10}$ gleich oder verschieden sind und bedeuten:
- ein Wasserstoffatom,
- eine Methylgruppe,
- eine tert.Butylgruppe.

6. Verfahren nach einem der vorangehenden Ansprüche, dadurch gekennzeichnet, daß der Ligand der in Anspruch 1 angegebenen Formel entspricht, wobei mindestens einer der Reste $R_1$ bis $R_3$ und mindestens einer der Reste $R_4$ bis $R_6$ Wasserstoffatome sind.

7. Verfahren nach einem der vorangehenden Ansprüche, dadurch gekennzeichnet, daß der Ligand der in Anspruch 1 angegebenen Formel entspricht, wobei mindestens zwei der Reste $R_1$ bis $R_3$ und mindestens zwei der Reste $R_4$ bis $R_6$ Wasserstoffatome sind.

8. Verfahren nach einem der vorangehenden Ansprüche, dadurch gekennzeichnet, daß der Ligand der in Anspruch 1 angegebenen Formel entspricht, wobei einer der Reste $R_1$ bis $R_3$ und einer der Reste $R_4$ bis $R_6$ eine Methylgruppe bedeutet oder $R_2$ und $R_5$ eine tert.Butylgruppe bedeuten und die anderen Reste $R_1$ und $R_6$ Wasserstoffatome bedeuten.

9. Verfahren nach Anspruch 8, dadurch gekennzeichnet, daß der Ligand ausgewählt ist aus Bis-1,3-(2-pyridyl-imino)isoindolin und Bis-1,3-(3-methyl-2-pyridyl-imino)isoindolin.

10. Verfahren nach einem der (vorausgehenden) Ansprüche, dadurch gekennzeichnet, daß das Ruthenium in das Reaktionsmedium in Form einer Verbindung eingebracht wird, ausgewählt aus Ruthenium-naphthenat, -octoat, -hexanoat und -acetylacetonat.

11. Verfahren nach Anspruch 10, dadurch gekennzeichnet, daß das Ruthenium in das Reaktionsmedium eingebracht wird in Form von Ruthenium-acetylacetonat.

12. Verfahren nach einem der vorangehenden Ansprüche, dadurch gekennzeichnet, daß die Zersetzung bei einer Temperatur zwischen 80 und 100 °C durchgeführt wird.

13. Verfahren nach einem der vorangehenden Ansprüche, dadurch gekennzeichnet, daß während der Zersetzung die Konzentration an Cyclohexyl-hydroperoxid zwischen 0,5 und 8 Gew.-% liegt.